# EUROPEAN PATENT APPLICATION

(11) **EP 2 799 106 A1**
(43) Date of publication of application: **05.11.2014**
(21) Application number: 14153161.6
(22) Date of filing: 30.01.2014
(51) Int. Cl.: A61M 25/09

(54) **Guidewire**

(30) Priority: 30.04.2013 JP 2013095170
(71) Applicant: ASAHI INTECC CO., LTD., Nagoya-shi, Aichi 463-0024 (JP)
(72) Inventor: Miyata, Naohiko c/o ASAHI INTECC CO., LTD., Nagoya-shi, Aichi 463-0024 (JP); Koike, Tadahiro c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft mbB

(57) **Abstract**

A coil-type guidewire (1, 2, 3, 4) includes a core shaft (10, 12, 14), a coil body (20, 22, 23, 24), and a plurality of bonding portions (30, 32, 34) that bond the core shaft (10, 12, 14) and the coil body (20, 22, 23, 24) to each other. At least one loosely wound coil portion is provided in at least one of regions sectioned by the bonding portions (30, 32, 34), and densely wound coil portions (20a, 20b, 20h, 22a, 22b, 22d, 23a, 23b, 23d, 24a, 24b, 24d) are disposed near the bonding portions (30, 32, 34).

## Description

### BACKGROUND OF THE INVENTION

### 1. Field of the Invention

The present invention relates to a guidewire to be inserted into a blood vessel.

### 2. Description of the Related Art

Guidewires used to insert a catheter into a blood vessel are known. To insert a catheter into a blood vessel, first, a guidewire is inserted into the blood vessel and through a lesion. After that, the catheter is inserted along the guidewire. Thus, the guidewire functions as a guide for guiding the catheter to the lesion.

Such a guidewire generally includes a core shaft including a distal portion covered with a coil body (so-called coil-type guidewire). In a coil-type guidewire, the coil body and the core shaft are bonded to each other at a plurality of locations with an adhesive, such as solder.

It is necessary that, even if the guidewire is in a bent state in the blood vessel, when an operator rotates the guidewire at the proximal end of the guidewire, the rotation be reliably transmitted to the distal end of the guidewire (torque transmission performance be maintained). With regard to this point, as described in, for example, United States Patent Nos. 5,259,393 and 5,353,808, the coil body may be configured to include a portion in which the winding pitch is large (loosely wound coil portion). In such a case, wire portions of the coil body do not easily interfere with each other when the guidewire is bent in the blood vessel, and stretching of the guidewire in the blood vessel can be suppressed. As a result, the torque transmission performance can be maintained even when the guidewire is bent in the blood vessel.

However, the above-described guidewire according to the related art has a problem that sufficient flexibility of the guidewire cannot be ensured. More specifically, in the guidewires described in United States Patent Nos. 5,259,393 and 5,353,808, a loosely wound coil portion is arranged in the immediate vicinity of a bonding portion. Therefore, in the case where the bonding portion is made of a fluid adhesive, such as solder, the adhesive spreads over the loosely wound coil portion and the width of the bonding portion increases. As a result, the flexibility of the guidewire decreases.

### SUMMARY OF THE INVENTION

The present invention has been made in light of the above-described problem of the related art, and an object of the present invention is to provide a coil-type guidewire that has sufficient torque transmission performance in a bent state and with which sufficient flexibility can be ensured.

To achieve the above-described object, a guidewire according to an aspect of the present invention has the following structure. That is, the guidewire according to the aspect of the present invention includes a core shaft, a coil body that covers the core shaft, and a plurality of bonding portions that bond the core shaft and the coil body to each other. The coil body includes at least one loosely wound coil portion in which the coil body has a large winding pitch and which is disposed in at least one of regions sectioned by the bonding portions, and densely wound coil portions in which wire portions of the coil body are in contact with each other and which are disposed near the bonding portions at both ends of the at least one of the regions in which the at least one loosely wound coil portion is provided.

In the guidewire according to the aspect of the present invention, the coil body includes the loosely wound coil portion. Therefore, when the guidewire is bent in a blood vessel, interference between the wire portions of the coil body does not easily occur. Therefore, stretching of the guidewire in the blood vessel can be suppressed, and the torque transmission performance of the guidewire can be maintained even when the guidewire is bent in the blood vessel.

In addition, in the guidewire according to the embodiment of the present invention, the densely wound coil portions are disposed near the bonding portions. Therefore, when the bonding portions are formed of a fluid adhesive, such as solder, spreading of the adhesive can be suppressed. As a result, the risk that the width of the bonding portions will be excessively large and the flexibility of the guidewire will be reduced can be reduced.

### BRIEF DESCRIPTION OF THE DRAWINGS

Fig. 1 is a diagram illustrating the structure of a guidewire according to a first embodiment of the present invention.
Fig. 2 is an enlarged sectional view of a distal portion of the guidewire according to the first embodiment of the present invention.
Fig. 3 is an enlarged sectional view of a distal portion of a guidewire according to a second embodiment of the present invention.
Fig. 4 is an enlarged sectional view of a distal portion of a guidewire according to a third embodiment of the present invention.
Fig. 5 is an enlarged sectional view of a distal portion of a guidewire according to a fourth embodiment of the present invention.

### DESCRIPTION OF THE PREFERRED EMBODIMENTS

### A. First Embodiment

To clarify the above-described features of the present invention, guidewires according to embodiments of the present invention will now be described.

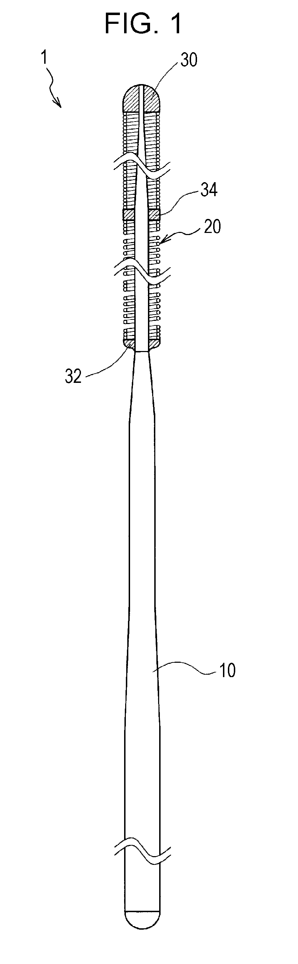

Fig. 1 is a diagram illustrating the structure of a guidewire 1 according to a first embodiment of the present invention. As illustrated in Fig. 1, the guidewire 1 according to the present embodiment includes a core shaft 10 and a coil body 20 arranged so as to cover a distal portion of the core shaft 10.

The core shaft 10 and the coil body 20 are bonded to each other with an adhesive (solder in the present embodiment). In the guidewire 1 according to the present embodiment, the core shaft 10 and the coil body 20 are bonded to each other with solder at a distal portion, a proximal portion, and an intermediate portion of the coil body 20. In the following description, a bonding portion for bonding the core shaft 10 to the distal portion of the coil body 20 is referred to as a distal bonding portion 30. A bonding portion for bonding the core shaft 10 to the proximal portion of the coil body 20 is referred to as a proximal bonding portion 32. A bonding portion between the distal bonding portion 30 and the proximal bonding portion 32 is referred to as an intermediate bonding portion 34.

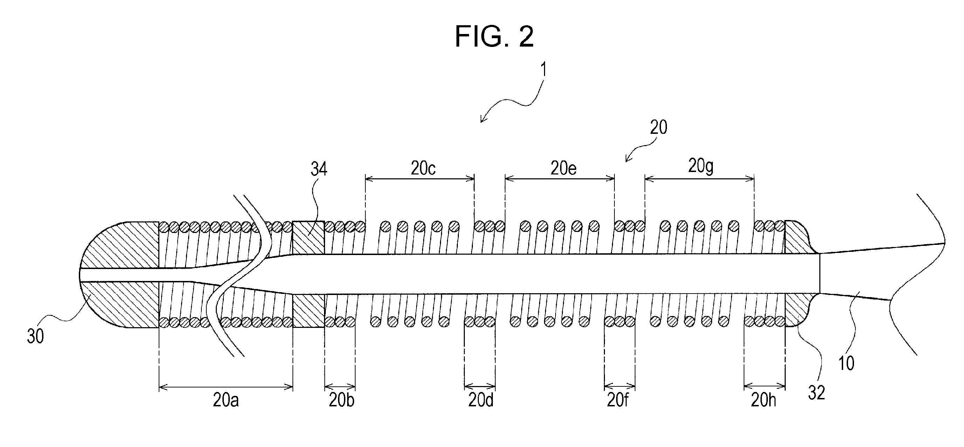

Fig. 2 is an enlarged sectional view of a distal portion of the guidewire 1 according to the first embodiment of the present invention. As illustrated in Fig. 2, in the guidewire 1 according to the present embodiment, a densely wound coil portion 20a is disposed in the region between the distal bonding portion 30 and the intermediate bonding portion 34. Here, the densely wound coil portion is a portion in which the winding pitch of the coil body 20 is very small and wire portions of the coil body 20 are in contact with each other.

A plurality of loosely wound coil portions 20c, 20e, and 20g (three loosely wound coil portions in the present embodiment) are arranged in the region between the intermediate bonding portion 34 and the proximal bonding portion 32. The loosely wound coil portions are portions in which the winding pitch of the coil body 20 is relatively large and wire portions of the coil body 20 are not in contact with each other.

Portions other than the loosely wound coil portions 20c, 20e, and 20g in the region between the intermediate bonding portion 34 and the proximal bonding portion 32 are formed as densely wound coil portions 20b, 20d, 20f, and 20h.

In the guidewire 1 according to the present embodiment, the densely wound coil portions 20b and 20h are disposed near the bonding portions at both ends of the region in which the loosely wound coil portions 20c, 20e, and 20g are arranged (the intermediate bonding portion 34 and the proximal bonding portion 32).

In the guidewire 1 according to the present embodiment, the loosely wound coil portions 20c, 20e, and 20g are arranged in at least one of the regions sectioned by the bonding portions (in the region between the intermediate bonding portion 34 and the proximal bonding portion 32 in the present embodiment). Therefore, when the guidewire 1 is bent in a blood vessel, the wire portions of the coil body 20 do not easily interfere with each other. As a result, stretching of the guidewire 1 in the blood vessel can be suppressed, and the torque transmission performance of the guidewire 1 can be maintained.

In the guidewire 1 according to the present embodiment, the densely wound coil portions 20a, 20b, and 20h are disposed near the intermediate bonding portion 34 and the proximal bonding portion 32. Therefore, when the intermediate bonding portion 34 (or the proximal bonding portion 32) is formed by using solder, spreading of the solder can be suppressed. As a result, the risk that the width of the intermediate bonding portion 34 (or the proximal bonding portion 32) will be excessively large and the flexibility of the guidewire 1 will be reduced can be reduced.

There are other embodiments that relate to the first embodiment. The other embodiments will now be briefly described. In the following description, components having the same structures as those of the components of the guidewire 1 according to the first embodiment are denoted by the same reference numerals, and detailed explanations thereof are thus omitted.

### B. Second Embodiment

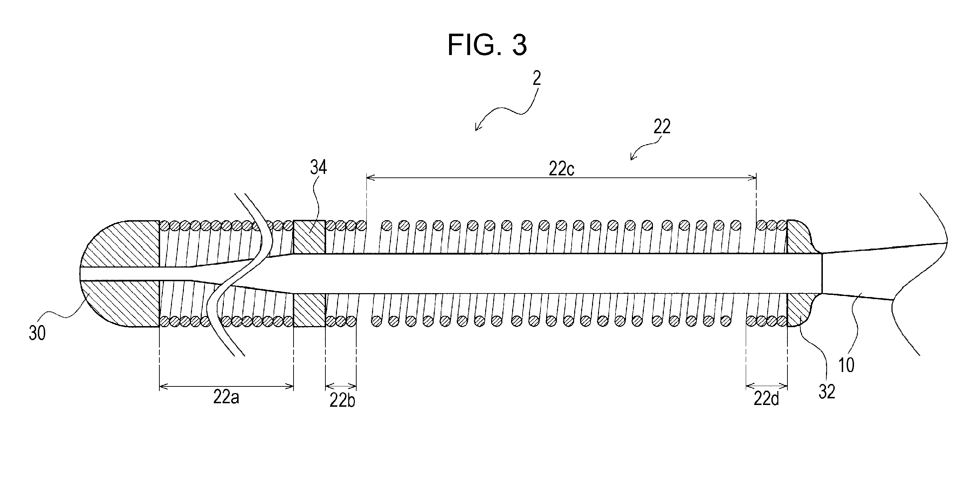

Fig. 3 is an enlarged sectional view of a distal portion of a guidewire 2 according to a second embodiment of the present invention. In the above-described guidewire 1 according to the first embodiment, the loosely wound coil portions 20c, 20e, and 20g are arranged with intervals therebetween in the region between the intermediate bonding portion 34 and the proximal bonding portion 32 (see Fig. 2). In contrast, in the guidewire 2 according to the second embodiment, a loosely wound coil portion 22c is disposed so as to extend over the entire region between densely wound coil portions 22b and 22d disposed at the distal end and the proximal end, respectively, of the region between the intermediate bonding portion 34 and the proximal bonding portion 32.

A densely wound coil portion 22a is disposed in the region between the distal bonding portion 30 and the intermediate bonding portion 34.

In the guidewire 2 according to the second embodiment, the loosely wound coil portion 22c is disposed so as to extend over substantially the entire region between the intermediate bonding portion 34 and the proximal bonding portion 32. Therefore, when the guidewire 2 is bent in a blood vessel, interference between wire portions of a coil body 22 can be reliably suppressed. As a result, stretching of the guidewire 2 in the blood vessel can be reliably suppressed, and the torque transmission performance of the guidewire 2 that is bent in the blood vessel can be reliably maintained.

### C. Third Embodiment

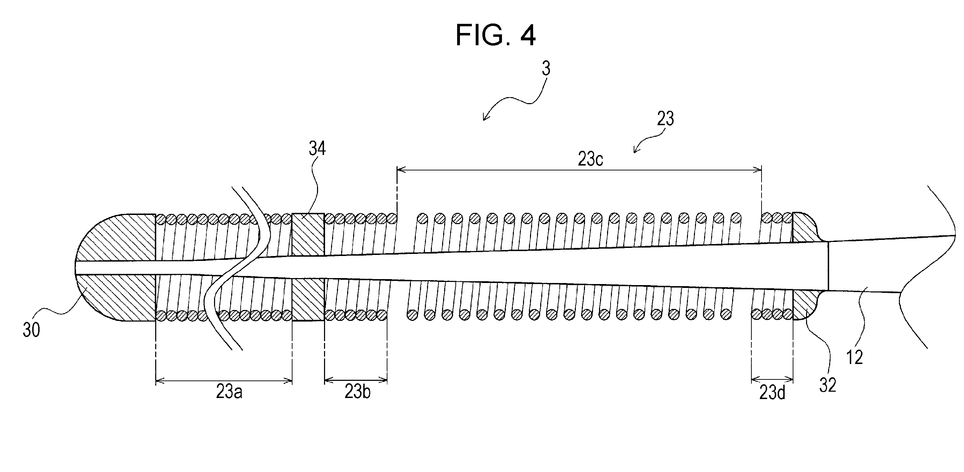

Fig. 4 is an enlarged sectional view of a distal portion of a guidewire 3 according to a third embodiment of the present invention. The structure of the guidewire 3 according to the third embodiment illustrated in Fig. 4 is similar to that of the above-described guidewire 2 according to the second embodiment except for the following two points.

That is, in the guidewire 2 according to the second embodiment, the core shaft 10 has a constant diameter in the region between the intermediate bonding portion 34 and the proximal bonding portion 32 (see Fig. 3). In contrast, in the guidewire 3 according to the third embodiment, a core shaft 12 is shaped such that the diameter thereof decreases toward the distal end (in other words, the core shaft 12 is tapered) in the region between the intermediate bonding portion 34 and the proximal bonding portion 32.

In addition, in the guidewire 2 according to the second embodiment, the densely wound coil portions 22b and 22d disposed at the distal end and the proximal end, respectively, of the region between the intermediate bonding portion 34 and the proximal bonding portion 32 have the same length (see Fig. 3). In contrast, in the guidewire 3 according to the third embodiment, a densely wound coil portion 23b disposed at the distal end of the region between the intermediate bonding portion 34 and the proximal bonding portion 32 is longer than a densely wound coil portion 23d disposed at the proximal end of the region between the intermediate bonding portion 34 and the proximal bonding portion 32.

A loosely wound coil portion 23c is provided between the densely wound coil portions 23b and 23d. A densely wound coil portion 23a is disposed in the region between the distal bonding portion 30 and the intermediate bonding portion 34.

With the guidewire 3 according to the third embodiment, the risk that the width of the bonding portions will be excessively large can be more reliably reduced. The reason for this will now be described.

In the case where the core shaft 12 is shaped such that the diameter thereof decreases toward the distal end as in the present embodiment, the gap between the inner peripheral surface of a coil body 23 and the outer peripheral surface of the core shaft 12 gradually increases toward the distal end. Therefore, a larger amount of adhesive is used at a distal bonding position than at a proximal bonding position.

In the present embodiment, as described above, the densely wound coil portion 23b at the distal end of the region between the intermediate bonding portion 34 and the proximal bonding portion 32 is longer than the densely wound coil portion 23d at the proximal end of the region between the intermediate bonding portion 34 and the proximal bonding portion 32. Therefore, even when a large amount of adhesive is used at the distal bonding position, spreading of the adhesive can be reliably suppressed. As a result, the risk that the width of a distal-side bonding portion (the intermediate bonding portion 34 in this case) will be excessively large can be reliably reduced.

As described above, with the guidewire 3 according to the third embodiment, the risk that the width of the bonding portions will be excessively large can be reliably reduced. Therefore, the risk that the flexibility of the guidewire 3 will be reduced can also be reliably reduced.

### D. Fourth Embodiment

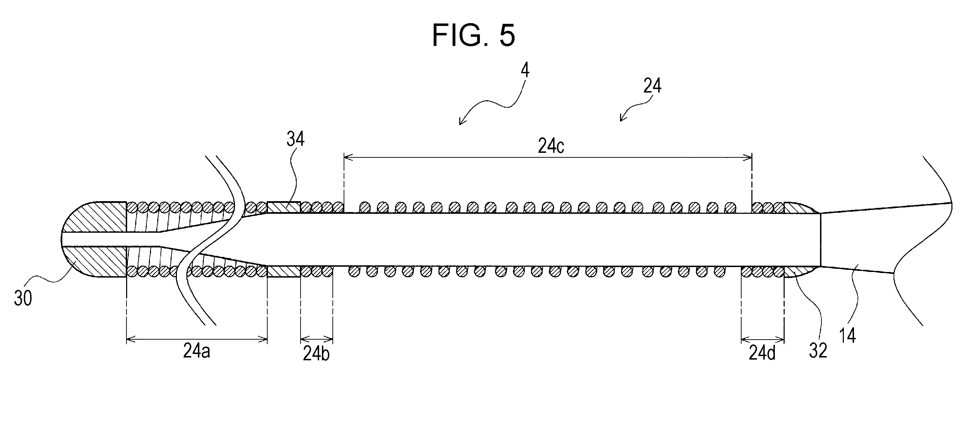

Fig. 5 is an enlarged sectional view of a distal portion of a guidewire 4 according to a fourth embodiment of the present invention. In the guidewire 4 according to the present embodiment, an inner peripheral surface of a coil body 24 and an outer coil body of a core shaft 14 are in contact with each other in the region between the intermediate bonding portion 34 and the proximal bonding portion 32. In this regard, the fourth embodiment differs from the guidewires 1 to 3 according to the above-described embodiments (see Figs. 2 to 4).

Also in the guidewire 4 according to the present embodiment, the coil body 24 includes a loosely wound coil portion 24c. Therefore, interference between wire portions of the coil body 24 can be suppressed when the guidewire 4 is bent in a blood vessel, and the torque transmission performance of the guidewire 4 can be maintained. In addition, since densely wound coil portions 24a, 24b, and 24d are disposed near the intermediate bonding portion 34 and the proximal bonding portion 32, when the intermediate bonding portion 34 (or the proximal bonding portion 32) is formed, the risk that the width of the intermediate bonding portion 34 (or the proximal bonding portion 32) will be excessively large and the flexibility of the guidewire 4 will be reduced can be reduced.

From the viewpoint of "maintaining the torque transmission performance of the guidewire in a bent state", a gap is preferably provided between the inner peripheral surface of the coil body and the outer peripheral surface of the core shaft as in the guidewires 1 to 3 of the above-described embodiments (see Figs. 2 to 4). In such a case, when the guidewire is bent in a blood vessel, interference between the coil body and the core shaft can be suppressed. For this reason and also because the interference between the wire portions of the coil body is also suppressed, the torque transmission performance of the guidewire in a bent state can be reliably maintained.

Although the guidewires according to the embodiments are described above, the present invention is not limited to the above-described embodiments, and various embodiments are possible within the scope of the present invention. For example, in the guidewires 1 to 4 according to the above-described embodiments, one or more loosely wound coil portions are provided in one of the regions sectioned by the bonding portions (region between the intermediate bonding portion 34 and the proximal bonding portion 32), and densely wound coil portions are disposed at both ends of that region. However, the loosely wound coil portions may be provided in all of the regions sectioned by the bonding portions, and the densely wound coil portions may be disposed near all of the bonding portions (not illustrated).

In this case, interference between the wire portions of the coil body can be suppressed irrespective of the position at which the guidewire is bent, and the torque transmission performance of the guidewire in a bent state can be more reliably maintained. In addition, when the densely wound coil portions are disposed near all of the bonding portions, the risk that the width of the bonding portions will be excessively large can be reduced for all of the bonding portions. Therefore, the flexibility of the guidewire can be increased.

In addition, in the guidewires 1 to 4 according to the above-described embodiments, the coil body and the core shaft are bonded to each other at three locations (the distal bonding portion 30, the intermediate bonding portion 34, and the proximal bonding portion 32). However, the coil body and the core shaft may instead be bonded to each other at a larger number of locations (four or more locations) (not illustrated). In such a case, the coil body and the core shaft can be more strongly bonded to each other.

## Claims

1. A guidewire (1, 2, 3, 4) comprising:
a core shaft (10, 12, 14);
a coil body (20, 22, 23, 24) that covers the core shaft (10, 12, 14); and
a plurality of bonding portions (30, 32, 34) that bond the core shaft (10, 12, 14) and the coil body (20, 22, 23, 24) to each other,
wherein the coil body (20, 22, 23, 24) includes
at least one loosely wound coil portion (20c, 20e, 20g, 22c, 23c, 24c) in which the coil body (20, 22, 23, 24) has a large winding pitch and which is disposed in at least one of regions sectioned by the bonding portions (30, 32, 34), and
densely wound coil portions (20a, 20b, 20h, 22a, 22b, 22d, 23a, 23b, 23d, 24a, 24b, 24d) in which wire portions of the coil body (20, 22, 23, 24) are in contact with each other and which are disposed near the bonding portions (30, 32, 34) at both ends of the at least one of the regions in which the at least one loosely wound coil portion is provided.

2. The guidewire according to Claim 1,
wherein the at least one loosely wound coil portion extends over the entire region between the densely wound coil portions disposed near distal and proximal ends of the at least one of the regions sectioned by the bonding portions (30, 32, 34).

3. The guidewire according to Claim 2,
wherein the core shaft is shaped such that a diameter thereof decreases toward a distal end thereof, and
wherein the densely wound coil portion disposed near the distal end of the at least one of the regions sectioned by the bonding portions (30, 32, 34) is longer than the densely wound coil portion disposed near at the proximal end of the at least one of the regions sectioned by the bonding portions (30, 32, 34).

4. The guidewire according to any one of Claims 1 to 3,
wherein a gap is provided between an inner peripheral surface of the coil body and an outer peripheral surface of the core shaft.
